# EUROPEAN PATENT APPLICATION

(11) **EP 4 656 163 A2**
(43) Date of publication of application: **03.12.2025**
(21) Application number: 25202677.8
(22) Date of filing: 11.11.2021
(51) Int. Cl.: A61F 2/38

(54) **KNEE IMPLANT**

(30) Priority: 14.12.2020 GB 202019691
(62) Divisional of application: 21815593.5
(71) Applicant: Invibio Knees Limited, Thornton Cleveleys, Lancashire FY5 4QD (GB)
(72) Inventor: REVIE, Ian, Thornton Cleveleys, FY5 4QD (GB); BRISCOE, Adam, Thornton Cleveleys, FY5 4QD (GB); WATKINS, Neil, Thornton Cleveleys, FY5 4QD (GB); THOMPSON, Nicola, Thornton Cleveleys, FY5 4QD (GB); BRADY, Mark, Thornton Cleveleys, FY5 4QD (GB)
(74) Representative: Thompson, Nicola Ruth

(57) **Abstract**

The invention relates to a medical implant comprising an implant body comprising a polymer material, said implant body having a bone-facing interface and an articulating interface, wherein a stiffening layer is provided over at least a portion of the bone-facing interface, and wherein the stiffening layer comprises a mesh formed of a polymer.

## Description

### BACKGROUND

The present invention relates to a medical device. In particular, the invention relates to an orthopaedic implant, for example a knee implant.

Prosthetic implants can be used to partially or completely replace diseased and/or damaged joint tissue. Such implants may include articulating surfaces that replace the bone's natural articulating surfaces. For example, an implant for a knee replacement may include a femoral implant and/or a tibial implant. A femoral implant may be implanted on the distal end of the femur and may replace the articulating surfaces of the femur. A tibial implant may be implanted on the proximal end of the tibia and may replace the articulating surfaces of the tibia. In operation, the articulating surfaces of the femoral implant articulate against the articulating surfaces of the tibial implant.

Various materials have been used for the femoral and tibial implants. For example, the implants may be made from metal, for instance, cobalt-chrome. Metal implants may have a significantly higher stiffness and tensile strength than bone. As a result, metal implants can have an increased tendency to shield the underlying bone from stresses that would normally be applied to the joint during use. According to Wolff's law, bone remodels in response to applied loads. If a bone is shielded from these loads, the bone will not be exposed to the stimulus required to maintain bone mass. This can lead to a loss of bone mass that may e.g. increase the chances of the implant coming loose. In recent years, there has been increasing interest in knee implants formed from polymeric compositions that may be formulated to have mechanical properties that are more compatible with the mechanical properties of bone.

To implant a femoral implant onto the distal femoral bone, the bone may need to be resected. The implant may be held in position using a bone cement, e.g. polymethylmethacrylate (PMMA). This type of fixation requires a mechanical bond to be formed between the cement and the bone, as well as a mechanical bond between the cement and the implant. In some instances, large loads transferred through the implant can cause one, or both, of these mechanical bonds to degrade, particularly over time. Furthermore, some patients may have adverse reactions to the bone cement.

Cementless implants may be used. Primary fixation of cementless implants may be obtained by the formation of compressive forces and subsequent shearing forces at the bone-implant interface during implantation. These compressive and shearing forces can be influenced by, for example, the interference fit and the frictional properties of the implant surface. The interference fit refers to the dimensional difference between the prepared bone cuts and the implant. The femoral bone may be cut slightly larger than the internal implant dimensions, resulting in a press fixation after implantation. The coefficient of friction may be altered by the roughness of the implant surface. In combination with the press-fit compressive forces, the roughness of the interface affects the resistance to shear forces at the implant-bone interface and can play an important role in the primary fixation of the implant. The roughness of the interface may also provide a textured surface that permits bone in-growth into the implant (secondary fixation).

### BRIEF DESCRIPTION OF THE DRAWINGS

Embodiments of the present invention are described, by way of example, with reference to the following figures, in which:

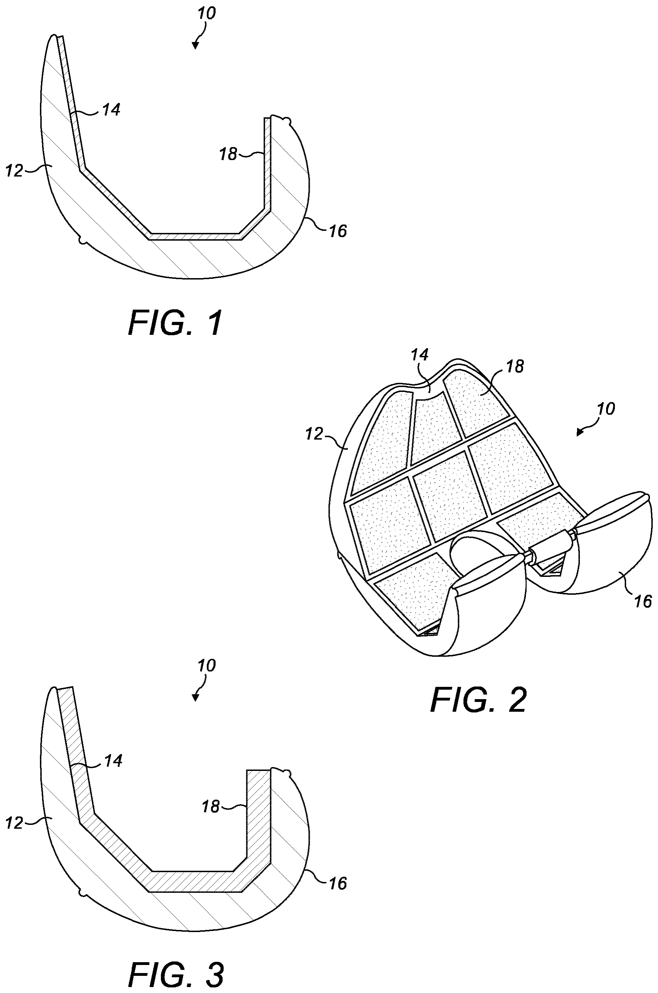

Figures 1 to 14 are schematic illustrations of examples of knee implants according to the present disclosure.

### DESCRIPTION

According to a first aspect of the present disclosure, there is provided a medical implant, more preferably, a knee implant comprising an implant body comprising a polymer material. The implant body has a bone-facing interface and an articulating interface. A stiffening layer is provided over at least a portion of the bone-facing interface. The stiffening layer is porous and is formed of a material having a higher stiffness than the polymer material of the underlying bone-facing interface. Preferably, the polymer material comprises a polyaryletherketone, more preferably, polyetheretherketone (PEEK).

By using a polymer material (e.g. polyaryletherketone), the implant may be formed having improved mechanical compatibility with bone. Accordingly, when the implant is implanted into bone, stress-shielding may be reduced and the distribution of load improved compared, for example, to metal implants. This can provide the underlying bone with sufficient biological stimulus for maintaining bone mass and/or reducing bone loss.

It has been found that, after implantation, the implant may move relative to the underlying bone (micromotion). While excessive micromotion (e.g. greater than 150 microns) may be undesirable, smaller micromotions (e.g. 40 microns or less) may be desirable to promote bone in-growth. Accordingly, to tailor the mechanical properties of at the bone-engaging surface to optimise bone in-growth, a porous stiffening layer is provided over at least a portion of the bone-facing interface of the implant. The pores in the stiffening layer may provide openings for bone in-growth at the implant/bone interface, facilitating fixation of the implant at the site of implantation. Such cementless fixation at the bone/implant interface may offer improved load transfer across the interface and into the e.g. femoral and/or tibial bone. In the present disclosure, the stiffening layer is also formed of a material having a higher stiffness than the polymer material of the implant body. This increased stiffness allows micromotions at the bone interface to be controlled to levels that improve bone-ingrowth, e.g. while reducing the risk of excessive micromotion that may lead to e.g. implant loosening. The degree of micromotion can also be controlled to favour the growth of certain cells (e.g. bone cell growth) over the growth of other cells (e.g. fibrous tissue).

The stiffening layer may comprise a mesh. The mesh may comprise multiple overlying layers of mesh material. The openings of at least one layer of mesh material may be partially offset with respect to the openings of at least one other layer of mesh material to provide (e.g. tortuous) channels through the thickness of the mesh.

The mesh may be formed of metal, for example, titanium.

The mesh may be formed of a polymer, preferably a reinforced polymer, for example, a fibre reinforced polyaryletherketone.

The implant is preferably an orthopaedic implant, most preferably a knee implant, for instance, a femoral implant and/or a tibial implant. In some examples, the implant may be a patellar implant. The implant may be a hip prosthesis.

The stiffening layer may be provided over a selected region or selected regions of the bone-facing interface. Alternatively, the stiffening layer may be provided over substantially the entirety of the bone-facing interface.

The stiffening layer may be formed of a first layer of material having a first stiffness and a second layer of material having a second stiffness to provide differing areas of stiffness in the stiffening layer. The first layer of material and/or the second layer of material may be of a uniform thickness. Alternatively, the first layer of material and/or the second layer of material is of a non-uniform thickness. In one example, either the first layer or second layer of material is of a uniform thickness and the other layer of material is of a non-uniform thickness.

### Polyaryletherketone

Any suitable polymer may be used to form the implant body of the implant of the present disclosure. Preferably, the polymer is a polyaryletherketone.

Suitable polyaryl ether ketone may have repeating units of formula (I) below: 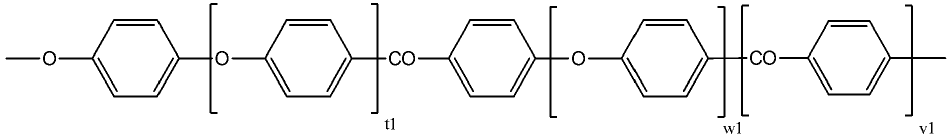 where t1 and w1 are independently represent 0 or 1 and v1 represents 0, 1 or 2.

The polyaryletherketone suitably includes at least 90, 95 or 99 mol % of repeat unit of formula I. The polyaryletherketone suitably includes at least 90, 95 or 99 weight % of repeat unit of formula I.

The polyaryletherketone may comprise or consist essentially of a repeat unit of formula I. Preferred polymeric materials comprise (or consist essentially of) a said repeat unit wherein t1=1 , v1=0 and w1=0; t1=0, v1=0 and w1=0; t1=0, w1 =1 , v1=2; or t1=0, v1=1 and w1=0. More preferably, the polyaryletherketone comprises (e.g. consists essentially of) the repeat unit I, wherein t1 =1 , v1 =0 and w1 =0; or t1=0, v1=0 and w1 =0. The most preferred polyaryletherketone comprises (especially consists essentially of) a said repeat unit wherein t1=1 , v1=0 and w1=0.

The polyaryletherketone may be selected from polyetheretherketone, polyetherketone, polyetherketoneetherketoneketone and polyetherketoneketone. In some examples, the polymer is selected from polyetherketone and polyetheretherketone. The polymer is preferably polyetheretherketone (PEEK).

The polyaryletherketone may have a Notched Izod Impact Strength (specimen 80mm x 10mm x 4mm with a cut 0.25mm notch (Type A), tested at 23°C, in accordance with ISO180) of at least 4 KJm⁻² , preferably at least 5 KJm⁻², more preferably at least 6 KJm⁻². The Notched Izod Impact Strength, measured as mentioned above, may be less than 10 KJm⁻², suitably less than 8 KJm⁻². The Notched Izod Impact Strength, measured as mentioned above, may be at least 3 KJm⁻², suitably at least 4 KJm⁻², preferably at least 5 KJm⁻². The impact strength may be less than 50 KJm⁻², suitably less than 30 KJm⁻².

The polyaryletherketone may have a melt viscosity (MV) of at least 0.06 kNsm⁻², preferably has a MV of at least 0.09 kNsm⁻², more preferably at least 0.12 kNsm⁻², especially at least 0.15 kNsm⁻². Advantageously, the MV may be at least 0.35 kNsm⁻² and especially at least 0.40 kNsm⁻². An MV of 0.45 kNsm⁻² may be particularly advantageous.

Unless otherwise stated, melt viscosity (MV) is measured using a Bohlin Instruments RH2000 capillary rheometer according to ISO 1 1443 operating at 340°C and a shear rate of 1000 s⁻¹ using a 0.5 mm (capillary diameter) x 8.0 mm (capillary length) die with entry angle 180°C. Granules may be loaded into the barrel and left to pre-heat for 10 minutes. The viscosity may be measured once steady state conditions are reached and maintained, nominally 5 minutes after the start of the test. The polyaryletherketone may have a MV of less than 1.00 kNsm⁻² , preferably less than 0.5 kNsm⁻² . The polyaryletherketone may have a MV in the range 0.09 to 0.5 kNsm⁻² , preferably in the range 0.14 to 0.5 kNsm⁻² , more preferably in the range 0.4 to 0.5 kNsm⁻² .

The polyaryletherketone may have a tensile strength, measured in accordance with IS0527 (specimen type 1 b) tested at 23°C at a rate of 50mm/minute of at least 20 MPa, preferably at least 60 MPa, more preferably at least 80 MPa. The tensile strength is preferably in the range 80-1 10 MPa, more preferably in the range 80-100 MPa.

The polyaryletherketone may have a flexural strength, measured in accordance with IS0178 (80mm x 10mm x 4mm specimen, tested in three-point-bend at 23°C at a rate of 2mm/minute) of at least 50 MPa, preferably at least 100 MPa, more preferably at least 145 MPa. The flexural strength is preferably in the range 145-180MPa, more preferably in the range 145-164 MPa. The polyaryletherketone may have a flexural modulus, measured in accordance with IS0178 (80mm x 10mm x 4mm specimen, tested in three-point-bend at 23°C at a rate of 2mm/minute) of at least 1 GPa, suitably at least 2 GPa, preferably at least 3 GPa, more preferably at least 3.5 GPa. The flexural modulus is preferably in the range 3.5-4.5 GPa, more preferably in the range 3.5-4.1 GPa.

The polyaryletherketone may be amorphous or semi-crystalline. The polyaryletherketone is preferably crystallisable. The polyaryletherketone may be semi-crystalline. The level and extent of crystallinity in a polymer may be measured by wide angle X-ray diffraction (also referred to as Wide Angle X-ray Scattering or WAXS), for example as described by Blundell and Osborn (Polymer 24, 953, 1983). Alternatively, crystallinity may be assessed by Differential Scanning Calorimetry (DSC).

The level of crystallinity of said polyaryletherketone may be at least 1 %, suitably at least 3%, preferably at least 5% and more preferably at least 10%. In especially preferred embodiments, the crystallinity may be greater than 25%. It may be less than 50% or less than 40%. The main peak of the melting endotherm (Tm) of said polyaryletherketone (if crystalline) may be at least 300°C.

### Implant Body

The polyaryletherketone described above may be used to form the implant body. For example, the polyaryletherketone described above may be used to form a polymer composition that is moulded to form the implant body. Examples of suitable moulding methods include injection moulding and compression moulding. Preferably, the implant body is formed by injection moulding.

In some examples, the polymer composition may include a contrast agent, for example, barium sulphate. For example, barium sulphate may be present in the polymer composition in an amount of 2 to 20 weight % of the total weight of the polymer composition, for instance, 3 to 10 weight %. In a preferred embodiment, the amount of barium sulphate may be 4 to 8 weight %, more preferably 4 to 6 weight %. In a most preferred embodiment, the amount of barium sulphate may be 5 weight %.

The implant body has a bone-facing interface and an articulating interface. The articulating interface may be considered as an external interface, while the bone-facing interface may be considered as an internal interface. The articulating interface may be smooth i.e. with a low coefficient of friction.

The implant body may form the body of a femoral implant or tibial implant. Preferably, the implant body may form the body of a femoral implant. In the case of a femoral implant, the articulating interface may be the interface that, during use, articulates against the articulating (bearing) interface of a tibial implant. The bone-facing interface may be the interface that is implanted to face the distal portion of the femur.

Where the implant body is the body of a femoral implant, the body of the femoral implant may comprise an external articulating interface. The outer articulating surface may comprise a first condyle and a second condyle. The first condyle and second condyle may correspond to the lateral and medial condyles of the femoral tissue of the original knee joint.

In some examples, an opening may be provided between the first condyle and second condyle. The opening may define an intercondylar notch. The intercondylar notch may be arranged to receive a stem provided on a corresponding tibial implant. This stem may restrict transverse movement of the femoral implant relative to the tibial implant.

Where the implant body is the body of a femoral implant, the body of the femoral implant may comprise an internal bone-facing interface. This bone-facing interface may be shaped to receive a distal portion of a femur. The bone-facing interface may comprise a bone-engaging protrusion (e.g. a stem) that extends from the bone-facing interface. Hole(s) may be made in the distal portion of the femur to receive such a bone-engaging protrusion to help secure the femoral implant in place. In some examples, the bone-facing interface may comprise two or more bone-engaging protrusions.

Where the implant body is the body of a tibial implant, the implant body may form the base plate of the tibial implant. The base plate may be shaped to accept a tibial articulating surface. This articulating surface ma engage a femoral component to form a knee joint. The distal section of the tibial baseplate may comprise a protrusion for extending into the underlying tibial bone to provide fixation. The bone-facing interface of the tibial implant may be shaped to receive a proximal portion of the tibia. The bone-facing interface may be provided with the stiffening layer.

### Stiffening Layer

As discussed above, a stiffening layer is provided over at least a portion of the bone-facing interface. The layer may be provided over substantially the whole surface of the bone-facing layer. The stiffening layer may be provided over between 1 to 100% of the surface area of the bone-facing interface. Preferably, said layer is provided over at least 90%, most preferably at least 95% of said interface. The layer may form a continuous or discontinuous area of said interface, for example, said layer may comprise a single piece, or may be provided as a plurality of individual pieces or strips, for example, dots on the interface.

The stiffening layer is porous and is formed of a material having a higher stiffness than the polymer material of the underlying bone-facing interface. The stiffening layer may also increase the coefficient of friction at the bone-facing interface. This may aid primary fixation of the implant. The preferred porosity of the stiffening layer is between 0.1mm to 10mm, more preferably between 0.1mm and 3mm, most preferably 0.15mm.

The stiffening layer may be formed by incorporating (e.g. embedding) reinforcement fibres (e.g. carbon fibres) into the polymer. The fibres may be disposed to provide a porosity, texture and increased stiffness to at least a portion of the bone-facing interface.

The stiffening layer and/or the implant body may comprise a bioactive material such as HA (hydroxyapatite) to improve bone fixation. Suitably, the bioactive material (II) comprises a material selected from the group consisting of apatites and calcium phosphates. Suitably, the bioactive material consists of an apatite and/or calcium phosphate. Suitably, the bioactive material consists of an apatite. Suitably, the bioactive material consists of hydroxyapatite (HA).

The stiffening layer may be moulded onto the bone-facing interface, for example, by injection or compression moulding. In some examples, the stiffening layer may be formed by 3D printing. The entire implant may be 3D printed to create a body having a first porosity and a stiffening layer having a second porosity. The stiffening layer may be integrally moulded to said interface or may be applied in a retrofit arrangement. The layer may be applied during or after the moulding process of the implant.

Preferably, the stiffening layer may comprise a mesh. The mesh may be formed of a metal. Any suitable biocompatible metal may be used. Suitable metals include stainless steel, cobalt-chrome, titanium and titanium alloy. Alternatively, the mesh may be formed of a polyaryletherketone, preferably a fibre reinforced PAEK, preferably a carbon fibre reinforced PEEK.

In some examples, the mesh may comprise multiple overlying layers of mesh material. The openings of at least one layer of mesh material may be partially offset with respect to the openings of at least one other layer of mesh material to provide channels through the thickness of the mesh. The offsetting may provide the channels with a degree of tortuosity. Such channels may promote bone in-growth through the stiffening layer.

The stiffness of the material of the stiffening layer may be selected to optimise the desired degree of micromotion of the implant relative to the underlying bone. The extent of micromotion should be sufficiently low to avoid significant risks of implant loosening. However, a degree of micromotion should be retained to promote bone in-growth.

The degree of micromotion may be less than 150 microns, preferably less than 120 microns, more preferably less than 100 microns, yet more preferably less than 80 microns. The degree of micromotion may be more than 10 microns, preferably more than 20 microns, more preferably more than 30 microns, yet more preferably more than 35 microns. In some examples, the degree of micromotion may be 10 to 150 microns, preferably, 20 to 120 microns, more preferably 30 to 100 microns, for example, 35 to 80 microns. In some examples, the degree of micromotion may be 40 to 60 microns or 40 to 50 microns.

Without wishing to be bound by any theory, it has been found that, as well as controlling the degree of micromotion to promote in-growth, the degree of micromotion may also control the type of cell in-growth into the stiffening layer. For example, by optimising the degree of micromotion below 120 microns, for example, from 20 to 100 microns, 30 to 80 microns, 35 to 60 microns or 40 to 50 microns, it may be possible to preferentially promote bone in-growth over e.g. fibrous tissue in-growth. This may improve fixation.

The stiffening layer may comprise substantially the whole of the implant. The layer may comprise up to 95% of the implant body preferably being a metal mesh having a thin PAEK veneer. The stiffening layer or the implant body has a minimum thickness of 0.5mm. The porosity of the mesh is preferably between 0.1mm to 10mm, most preferably 0.15mm.

As well as varying the stiffness of the material used to form the stiffening layer, it may also be possible to control the degree of micromotion by varying the extent and nature of coverage of the stiffening layer over the bone-facing interface. For example, the stiffening layer may be provided over a selected region or selected regions of the bone-facing interface. Alternatively, the stiffening layer may be provided over substantially the entirety of the bone-facing interface.

It may also be possible to vary the stiffness profile of the stiffening layer or to vary the stiffness of the stiffening layer over different regions of the bone-facing interface. For example, the stiffening layer may be formed of a first layer of material having a first stiffness and a second layer of material having a second stiffness to provide differing areas of stiffness in the stiffening layer. The first layer of material and/or the second layer of material may be of a uniform thickness. Alternatively, the first layer of material and/or the second layer of material is of a non-uniform thickness. In one example, either the first layer or second layer of material is of a uniform thickness and the other layer of material is of a non-uniform thickness.

These and other aspects of the present invention will now be described with reference to the accompanying drawings.

Referring to Figure 1, this is a schematic cross-sectional view of a femoral implant 10. The femoral implant 10 comprises an implant body 12 comprising a polymer material comprising polyaryletherketone (e.g. PEEK). The implant body 12 comprises a bone-facing interface 14 and an articulating interface 16. A stiffening layer 18 is provided over at least a portion of the bone-facing interface 14. The stiffening layer 18 is porous and is formed of a material having a higher stiffness than the polymer material of the implant body 12. In the example of Figure 1, the stiffening layer 18 is formed from a mesh comprising layers of overlying mesh material (e.g. titanium wire mesh material). In Figure 1, the stiffening layer 18 is of uniform thickness and lines substantially the entire bone-facing interface 14.

Figure 2 is a schematic perspective view of a similar femoral implant 10 to that shown in Figure 1. In Figure 2, however, the stiffening layer 18 is applied to selected areas of the bone-facing interface.

Figure 3 is a schematic cross-sectional view of a similar femoral implant 10 to that shown in Figure 1. In Figure 3, however, the thickness of the stiffening layer 18 is greater than the thickness shown in Figure 1.

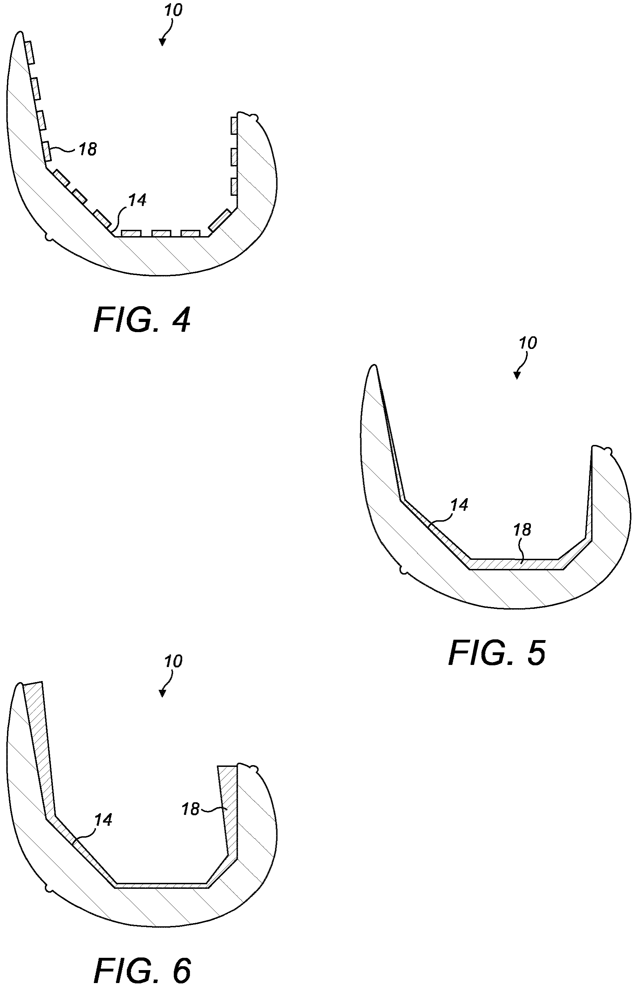

Figure 4 is a schematic cross-sectional view of a similar femoral implant 10 to that shown in Figure 1. In Figure 4, however, the stiffening layer 18 is applied discontinuously across the bone-facing interface 14. In this Figure, strips of stiffening layer 18 are applied at space intervals. This pattern, however, is merely illustrative and other discontinuous patterns may be employed.

Figure 5 is a schematic cross-sectional view of a similar femoral implant 10 to that shown in Figure 1. In Figure 5, however, the stiffening layer 18 is applied in varying thickness over the bone-facing interface. As illustrated in Figure 5, the stiffening layer 18 in the proximal region is thinner than the stiffening layer 18 in the distal region. Conversely, in the embodiment shown in Figure 6, the stiffening layer 18 in the proximal region is thicker than the stiffening layer 18 in the distal region. These thickness patterns are merely illustrative and other thickness patterns may be employed.

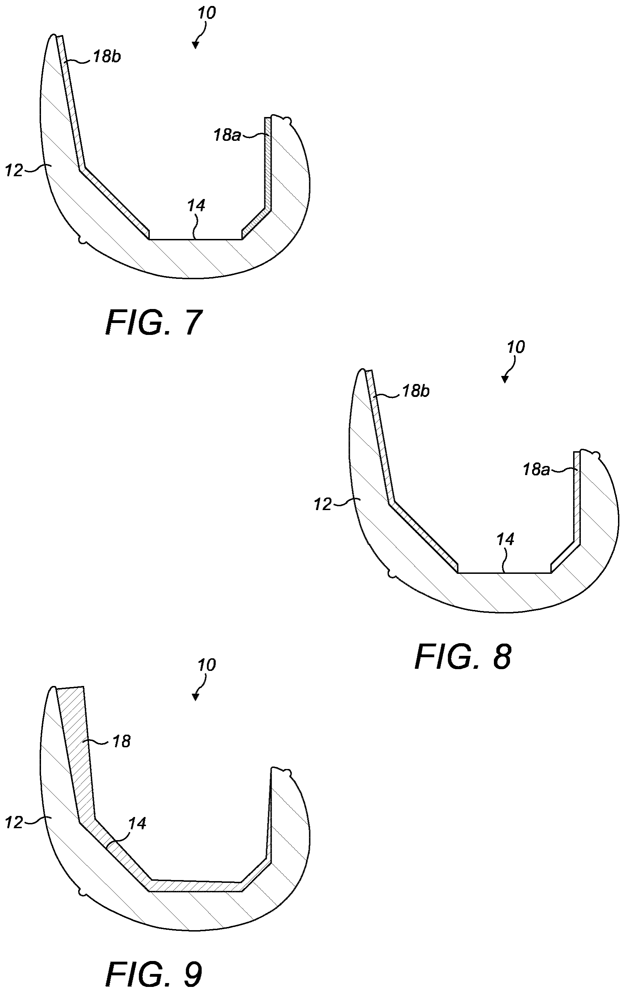

Figure 7 is a schematic cross-sectional view of a similar femoral implant 10 to that shown in Figure 1. In Figure 7, however, the stiffening layer 18 comprises a first stiffening layer portion 18a and a second stiffening layer portion 18b. The first stiffening layer portion 18a is placed over a posterior portion of the bone-facing interface 14 and the second stiffening layer portion 18b is placed over an anterior portion of the bone-facing interface. An intermediate distal portion of the bone-facing interface 14 may be left uncovered. The first stiffening layer portion 18a may have a different stiffness to the second stiffening layer portion 18b. In one example, the portion 18a may be stiffer than portion 18b. Alternatively, the portion 18a may be less stiff than portion 18b. In yet a further alternative, the first stiffening layer portion 18a and the second stiffening layer portion 18b may be formed of material having the same stiffness (see Figure 8).

Figure 9 shows an embodiment where the stiffening layer 18 has a higher thickness over an anterior portion of the bone-facing interface 14 than over a posterior portion of the bone-facing interface 14. The stiffening layer 18 may be positioned over substantially the entirety of the bone-facing interface 14.

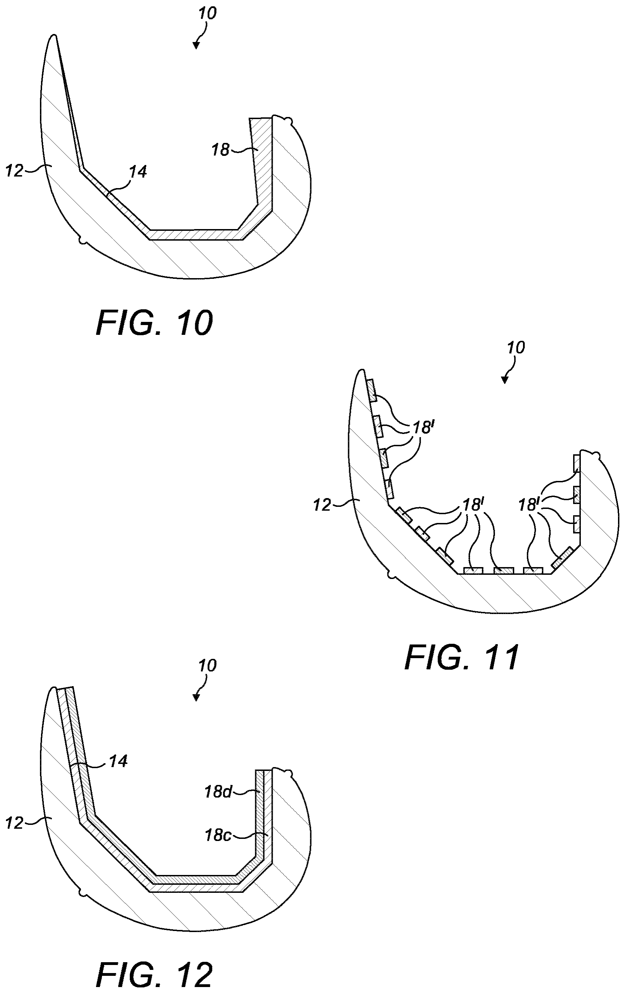

Figure 10 is an alternative to the embodiment of Figure 9, where the stiffening layer 18 has a lower thickness over an anterior portion of the bone-facing interface 14 than over a posterior portion of the bone-facing interface 14.

In Figure 11, the stiffening layer 18 comprises multiple strips of stiffening layer portions 18'. The stiffening layer portions 18' may have different stiffnesses. The stiffening layer portions 18' may have the same thickness. Alternatively, the stiffening layer portions 18' may have different thicknesses (not shown).

In Figure 12, the stiffening layer 18 comprises multiple layer portions, for example, first and second layer portions 18c and 18d. The layer portions 18c and 18d may overlie one another. The layer portions 18c and 18d may have different stiffnesses. This may provide a stiffness profile through the thickness of the stiffening layer 18.

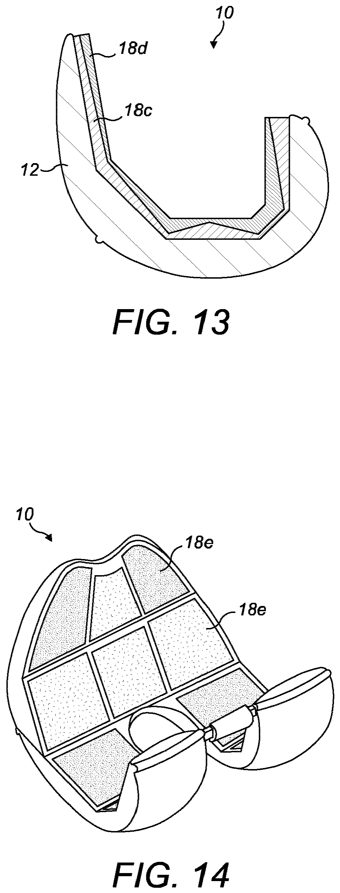

Figure 13 shows a variant of the embodiment shown in Figure 12. The layer portions 18c and 18d may each vary in thickness with the overall thickness of the stiffening layer 18 remaining constant.

In Figure 14, the stiffening layer 18 comprises multiple stiffening layer portions 18e positioned on selected portions of the bone-facing interface 14. The layer portions 18e may have different thicknesses and/or stiffnesses depending on the location of the layer portions 18e on the bone-facing interface 14.

Also disclosed are the following aspects of the invention which were set out in the claims of the parent application, European Application number 21815593.5, as follows:
1. A medical implant comprising an implant body comprising a polymer material, said implant body having a bone-facing interface and an articulating interface,
   wherein a stiffening layer is provided over at least a portion of the bone-facing interface, and
   wherein the stiffening layer is porous and is formed of a material having a higher stiffness than the polymer material of the underlying bone-facing interface.
2. An implant as claimed in claim 1, wherein the stiffening layer comprises a mesh.
3. An implant as claimed in claim 2, wherein the mesh comprises multiple overlying layers of mesh material.
4. An implant as claimed in claim 3, wherein the openings of at least one layer of mesh material are partially offset with respect to the openings of at least one other layer of mesh material to provide channels through the thickness of the mesh.
5. An implant as claimed in any one of claims 2 to 4, wherein the mesh is formed of metal.
6. An implant as claimed in any one of claims 2 to 4, wherein the mesh is formed from fibre-reinforced polyaryletherketone.
7. An implant as claimed in any one of the preceding claims that is a knee implant.
8. An implant as claimed in any one of the preceding claims, wherein the polymer comprises a polyaryletherketone.
9. An implant as claimed in claim 8, wherein the polyaryletherketone is PEEK.
10. An implant as claimed in any one of the preceding claims, wherein the stiffening layer is provided over a selected region or selected regions of the bone-facing interface.
11. An implant as claimed in any one of the preceding claims, wherein the stiffening layer is provided over substantially the entirety of the bone-facing interface.
12. An implant as claimed in any one of the preceding claims, wherein the stiffening layer is formed of a first layer of material having a first stiffness and a second layer of material having a second stiffness to provide differing areas of stiffness in the stiffening layer.
13. An implant as claimed in claim 12, wherein the first layer of material and/or the second layer of material is of a uniform thickness.
14. An implant as claimed in claim 12, wherein either the first layer or second layer of material is of a uniform thickness and the other layer of material is of a non-uniform thickness.
15. An implant as claimed in claim 12, wherein the first layer of material and/or the second layer of material is of a non-uniform thickness.

### Definitions

It is noted that, as used in this specification and the appended claims, the singular forms "a," "an," and "the" include plural referents unless the content clearly dictates otherwise.

As used herein, the term "about" is used to provide flexibility to a range endpoint by providing that a given value may be "a little above" or "a little below" the endpoint. The degree of flexibility of this term can be dictated by the particular variable and can be determined based on experience and the associated description herein.

As used herein, a plurality of items, structural elements, compositional elements, and/or materials may be presented in a common list for convenience. However, these lists should be construed as though each individual member of the list is individually identified as a separate and unique member. Thus, no individual member of such list should be construed as a de facto equivalent of any other member of the same list solely based on their presentation in a common group without indications to the contrary.

Concentrations, dimensions, amounts, and other numerical data may be presented herein in a range format. It is to be understood that such range format is used merely for convenience and brevity and should be interpreted flexibly to include the numerical values explicitly recited as the limits of the range, and also to include all the individual numerical values or sub-ranges encompassed within that range as if the numerical value and sub-range is recited. For example, a weight ratio range of about 1 wt% to about 20 wt% should be interpreted to include the explicitly recited limits of 1 wt% and about 20 wt%, and also to include individual weights such as 2 wt%, 11 wt%, 14 wt%, and sub-ranges such as 10 wt% to 20 wt%, 5 wt% to 15 wt%, etc.

## Claims

1. A medical implant comprising an implant body comprising a polymer material, said implant body having a bone-facing interface and an articulating interface, wherein a stiffening layer is provided over at least a portion of the bone-facing interface, and wherein the stiffening layer comprises a mesh formed of a polymer.

2. An implant as claimed in claim 1, wherein the mesh is formed from polyaryletherketone (PAEK).

3. An implant as claimed in any one of the preceding claims, wherein the mesh is formed from a fibre reinforced PAEK.

4. An implant as claimed in claim 3, wherein the mesh is formed from a carbon fibre reinforced PAEK.

5. An implant as claimed in any one of the preceding claims wherein the mesh comprises multiple overlying layers of mesh material.

6. An implant as claimed in any one of the preceding claims, wherein openings of at least one layer of mesh material are partially offset with respect to the openings of at least one other layer of mesh material to provide channels through the thickness of the mesh.

7. An implant as claimed in any one of the preceding claims, wherein the polyaryletherketone has a flexural modulus, measured in accordance with IS0178 (80mm x 10mm x 4mm specimen, tested in three-point-bend at 23°C at a rate of 2mm/minute) in the range 3.5-4.5 GPa.

8. An implant as claimed in any one of the preceding claims that is a femoral implant.

9. An implant as claimed in claims 1 to 7, wherein said implant is a tibial implant.

10. An implant as claimed in any one of the preceding claims, wherein the polymer material of the implant body comprises a polyaryletherketone.

11. An implant as claimed in any one of the preceding claims, wherein the stiffening layer is provided over substantially the entirety of the bone-facing interface.

12. An implant as claimed in any one of the preceding claims, wherein the stiffening layer is formed of a first layer of material having a first stiffness and a second layer of material having a second stiffness to provide differing areas of stiffness in the stiffening layer.

13. An implant as claimed in claim 12, wherein the first layer of material and/or the second layer of material is of a uniform thickness.

14. An implant as claimed in claim 12, wherein either the first layer or second layer of material is of a uniform thickness and the other layer of material is of a non-uniform thickness.

15. An implant as claimed in claim 12, wherein the first layer of material and/or the second layer of material is of a non-uniform thickness.
